# EUROPEAN PATENT APPLICATION

(11) **EP 4 542 231 A1**
(43) Date of publication of application: **23.04.2025**
(21) Application number: 23203835.6
(22) Date of filing: 16.10.2023
(51) Int. Cl.: G01N 33/574

(54) **METHODS FOR IDENTIFYING AND STRATIFYING CANCERS AND CANCER PATIENTS BASED ON P2X4 RECEPTOR EXPRESSION**

(71) Applicant: Johann-Wolfgang-Goethe-Universität Frankfurt am Main, 60323 Frankfurt am Main (DE); Chemotherapeutisches Forschungsinstitut Georg-Speyer-Haus, 60596 Frankfurt am Main (DE)
(72) Inventor: Greten, Florian, 61352 Bad Homburg (DE); Ziegler, Paul, 60528 Frankfurt am Main (DE)
(74) Representative: Krauss, Jan

(57) **Abstract**

The present invention relates to methods for identifying cancers, responders, predicting a response and stratifying patients with respect to a combination therapy comprising administration of P2X4 receptor inhibitors and cell death inducing chemotherapy. The method according to the present invention identifies cancers, responders, predicts responders and stratifies patients based on detecting the P2X4 receptor protein expression in cells of a patientderived cancer sample, such as a cell or organoid, wherein an increase in the protein expression identifies cancers, responders, and predicts responders when applying the combination therapy, and further allows to stratify the patients accordingly.

## Description

The present invention relates to methods for identifying cancers, responders, predicting a response and stratifying patients with respect to a combination therapy comprising administration of P2X4 receptor inhibitors and cell death inducing chemotherapy. The method according to the present invention identifies cancers, responders, predicts responders and stratifies patients based on detecting the P2X4 receptor protein expression in cells of a patient-derived cancer sample, such as a cell or organoid, wherein an increase in the protein expression identifies cancers, responders, and predicts responders when applying the combination therapy, and further allows to stratify the patients accordingly.

### Background of the invention

The timely and precise prediction and/or identification of cancer cells and responders to anti-cancer therapies and a respective patient stratification play important roles in clinical practice and drug development. For patients suffering from cancer, a fast diagnosis and respective stratification for an effective treatment is paramount to prevent cancer progression, and metastasis. Key issues associated with a response and stratification of cancer patients include the complexity and heterogeneity of the molecular background behind the specific cancer disease.

Solid cancers exhibit a dynamic balance between cell death and proliferation ensuring continuous tumour maintenance and growth. Many chemotherapies rely on promoting apoptosis to reduce tumor size. However, enhanced cancer cell apoptosis is found to trigger paracrine activation of cells in the tumour microenvironment initiating tissue repair programs that support tumour growth, thereby triggering insensitivity, if not resistance, to apoptosis-targeting chemotherapy.

On a molecular level, cell death leads to the release of damage-associated molecular patterns (DAMPs) including various growth factors, inflammatory mediators, mitochondrial DNA (mtDNA), reactive oxygen species (ROS) and metabolites that trigger inflammation and activate innate immunity to stop the damage and orchestrate tissue repair and wound healing.

Schmitt et al, (in "Colon tumour cell death causes mTOR dependence by paracrine P2X4 stimulation." Nature 612.7939 (2022): 347-353.), by studying Lgr5+ cells containing tumor organoids disclosed a molecular mechanism of how this paracrine activation by dying cancers cells potentially contributes to therapy resistance involving the mTOR dependent pro-survival program. Lgr5+ cells have been described as cell of origin and cancer stem cells in colorectal cancer (CRC). It suggests that tumor cells - when killed during chemo- and/or toxin therapy - release messenger compounds, in particular ATP, into the tumor environment that improve survival of the neighboring tumour cells based on the functions of mTOR and P2X4.

mTOR is a serine-threonine kinase involved in the PI3K/mTOR pathway that regulates cell growth and proliferation in response to the availability of growth factors and nutrients. And Schmitt et al suggests that extracellular ATP release, particularly those released from dying cancer cells leads to paracrine mTORC1 activation. The ATP-associated mTOR activation occurs via purinergic receptors of the P2 family, which comprises seven subtypes of P2X receptors and 8 (in mice 7) subtypes of P2Y receptors. Gene expression of all known subtypes in both human untransformed and tumor organoids as well as in murine tumor organoids and detected prominent expression of P2X4. Immunohistochemistry confirmed expression of P2X4 in unchallenged colon and colonic tumors. Additionally, selective P2X4 inhibition using 5-(3-Bromophenyl)-1,3-dihydro-2H-Benzofuro[3,2-e]-1,4-diazepin-2-one (5-BDBD)24 reduced ATP-induced S6 phosphorylation in PDTOs, whereas selective P2X1 (NF279) and P2X7 inhibitors (A-438079) had no effect. This evidence collectively indicates that P2X4 receptor plays a crucial role in the signaling pathway involved in mTOR-associated tumor cell survival.

EP21163448 discloses the use of inhibitors of P2X4 receptor protein or inhibitors of the P2X4 pathway in combination with cell death inducing therapy to sensitize tumors that are otherwise resistant to cell death inducing chemotherapy. However, the combination chemotherapy still lacks predictive biomarker for identifying sensitive cancers, responders and/or for stratifying patients.

Therefore, it is an object of the present invention to provide such a predictive biomarker for identifying sensitive cancers, responders and/or for stratifying patients to a combination therapy involving P2X4 receptor inhibitor and chemotherapy.

Surprisingly, the inventor of the present invention found that the expression level of the p2rx4, the gene coding for P2X4 receptor protein, is not significantly different between cancer types, making any transcriptomic analysis unsuitable for stratification (see example 1). In contrast, despite the negligible difference in the gene expression level, the protein level of the P2X4 receptor differs significantly between cancer subtypes (see example 2).

In line with this finding, the above object in a first aspect of the present invention is solved by providing a method for identifying a cancer cell as being sensitive to an anti-cancer chemotherapy comprising the administration of a combination of i) an effective amount of at least one inhibitor of the P2X4 receptor and/or inhibitor of the P2X4 receptor signaling pathway and ii) an effective amount of at least one cell death inducing chemotherapeutic drug and/or cell death inducing therapy, comprising the step of detecting P2X4 receptor protein expression in a cancer cell, wherein the detection of P2X4 receptor protein expression in the cancer cell when compared to a non-P2X4 receptor protein expressing cancer control cell identifies the cancer cell as being sensitive to said combination chemotherapy.

The above object in a second aspect of the present invention is further solved by providing a method for predicting the response of a patient to an anti-cancer chemotherapy comprising the administration of a combination of i) an effective amount of at least one inhibitor of the P2X4 receptor and/or inhibitor of the P2X4 receptor signaling pathway and ii) an effective amount of at least one cell death inducing chemotherapeutic drug and/or cell death inducing therapy, said method comprising the step of detecting the expression of P2X4 receptor protein in cancer cells in a cancer sample from said patient, wherein the detection of P2X4 receptor protein expression in the sample when compared to a non-P2X4 receptor protein expressing cancer control cell predicts a positive response of said patient to said combination chemotherapy.

The above object in a third aspect of the present invention is further solved by providing a method for identifying a responder to an anti-cancer chemotherapy, comprising the steps of detecting the expression of P2X4 receptor protein in cancer cells in a cancer sample obtained from a patient treated with an anti-cancer chemotherapy comprising the administration of a combination of i) an effective amount of at least one inhibitor of the P2X4 receptor and/or inhibitor of the P2X4 receptor signaling pathway and ii) an effective amount of at least one cell death inducing chemotherapeutic drug and/or cell death inducing therapy, wherein the detection of the P2X4 receptor protein expression in the sample when compared to a control cancer sample identifies the patient as a responder to said combination chemotherapy.

Further preferred is the method according to the present invention, further comprising the step of stratifying the patient into a treatment group for applying a suitable anti-cancer therapy, in particular a combination chemotherapy comprising the administration of a combination of i) an effective amount of at least one inhibitor of the P2X4 receptor and/or inhibitor of the P2X4 receptor signaling pathway and ii) an effective amount of at least one cell death inducing chemotherapeutic drug and/or cell death inducing therapy.

Further preferred is the method according to the present invention, wherein the detection of the P2X4 receptor protein expression comprises determining the protein expression of the P2X4 receptor protein in the cancer cell, a cancer tissue sample comprising the cell, an organoid comprising the cancer cell or other suitable cancer sample.

Further preferred is the method according to the present invention, wherein the detection comprises immunohistochemical analysis of P2X4 expression in a tissue or organoid.

The above object in a fourth aspect of the present invention is further solved by providing the use of a kit comprising a P2X4 receptor protein binding antibody or P2X4 receptor protein fragment or derivative thereof for identifying a cancer cell as being sensitive to an anti-cancer chemotherapy, for predicting the response of a patient to an anti-cancer chemotherapy, for identifying a responder to an anti-cancer chemotherapy and/or for stratifying a patient into a treatment group according to a method according to the present invention, and optionally further comprising materials to perform said method, such as buffers and reagents, as well as user instructions.

The above object in a fourth aspect of the present invention is further solved by providing a method for treating cancer, comprising the steps of performing the method according to the present invention, and administering a combination of i) an effective amount of at least one inhibitor of the P2X4 receptor and/or inhibitor of the P2X4 receptor signaling pathway and ii) an effective amount of at least one cell death inducing chemotherapeutic drug and/or cell death inducing therapy to the patient based on the result of the method according to the present invention.

As mentioned above, it was surprisingly found in the context of the present invention, that the expression of the P2X4 receptor protein differs significantly between cancer subtypes and can thus be used as a biomarker. In line with this finding, the present invention provides methods and devices for identifying cancers, responders, predicting a response and stratifying patients with respect to a combination therapy comprising administration of P2X4 receptor inhibitors and cell death inducing chemotherapy. The method according to the present invention identifies cancers, responders, predicts responders and stratifies patients based on detecting the P2X4 receptor protein expression in cells of a patient-derived cancer sample, such as a cell or organoid, wherein an increase in the protein expression identifies cancer cells, responders, and predicts responders when applying the combination therapy, and further allows to stratify the patients accordingly.

Therefore, the above object in a first aspect of the invention is solved by providing a method for identifying a cancer cell as being sensitive to an anti-cancer chemotherapy. The anti-cancer chemotherapy according to the present invention comprises the administration of a combination of i) an effective amount of at least one inhibitor of the P2X4 receptor and/or inhibitor of the P2X4 receptor signaling pathway and ii) an effective amount of at least one cell death inducing chemotherapeutic drug and/or cell death inducing therapy with that drug.

In general, any suitable at least one inhibitor of the P2X4 receptor and/or inhibitor of the P2X4 receptor signaling pathway may be used. Preferred is a method according to the present invention, wherein the inhibitor of the P2X4 receptor is a specific inhibitor of the P2X4 receptor, and is preferably selected from the group consisting of PPADS, Suramin, KN-62, TNP-ATP, Brilliant Blue G, 5-BDBD, BX-430, Carbamazepine der., PSB-12054, PSB-12062, PSB-15417, NP-1815-PX, NC-2600, UoS14919, Paroxetine, Duloxetine, BAY-1797, BAY-2328065, IgG#151-LO, an anti-P2X4 receptor protein antibody or an inhibitor binding fragment or derivative thereof, an siRNA, an shRNA, an miRNA, a ribozyme, an aptamer, an antisense nucleic acid molecule, and a small molecule of less that about 500 Da, preferably 5DBDB, and further preferred BAY-2328065.

Further preferred is a method according to the present invention, wherein the cell death inducing chemotherapeutic drug and/or cell death inducing therapy is selected from the group consisting of necrotic cell supernatants; cationic amphiphilic drugs (CAD); classical anticancer agents, including anthracyclines, antimetabolites, and platinum drugs; topoisomerase inhibitors, e.g. camptothecin; PDE3A inhibitors, such as anagrelide, either alone or with cell death-inducing cytokines; death-inducing cytokines, such as IFN-α, IFN-γ, TNF-α, or TRAIL; taxanes, paclitaxel, and fluorinated taxanes, such as SB-T-12851, SB-T-12852, SB-T-12853, SB-T-12854, 5-fluorouracil, tegafur, capecitabine, and doxifluridine; and preferably 5-fluorouracil (5-FU).

In the context of the present invention, an "effective amount" of the least one inhibitor of the P2X4 receptor and/or inhibitor of the P2X4 receptor signaling pathway and the at least one cell death inducing chemotherapeutic drug and/or cell death inducing therapy shall mean that the compound is provided in an amount to the patient, that a treatment or prevention with respect to the cancer as treated or prevented is achieved.

The method according to this embodiment then comprises the step of detecting the P2X4 receptor protein expression in a cancer cell. The detection of the P2X4 receptor protein expression comprises determining the protein expression of the P2X4 receptor protein in a sample derived from the patient, comprising the cancer cell, a cancer tissue sample comprising the cancer cell, or an organoid, for example an organoid obtained from an (earlier) patient sample, comprising the cancer cell. Also, any other suitable cancer sample as obtained from the patient may be used.

The patient may be any suitable cancer patient, such as, for example, a mammalian patient, such as a mouse, pet, cattle or human.

As described herein, the detection of P2X4 receptor protein expression in the cancer cell when compared to a non-P2X4 receptor protein expressing cancer control cell identifies the cancer cell as being sensitive to the combination chemotherapy as described herein, see also further below.

The methods according to the present invention may be performed in vivo or in vitro.

As mentioned above, it was furthermore surprisingly found in the context of the present invention, that the expression of the P2X4 receptor protein differs significantly between cancer subtypes in patients that are responders or non-responders to the anti-cancer chemotherapy as described herein. In line with this finding, the present invention provides a method for predicting the response of a patient to an anti-cancer chemotherapy as described herein. The method according to the present invention comprises the step of detecting the expression of P2X4 receptor protein in cancer cells in a cancer sample from said patient as described herein.

A detection of P2X4 receptor protein expression in the sample when compared to a non-P2X4 receptor protein expressing cancer control cell allows to predict a positive response of said patient to the present combination chemotherapy. In the context of the present invention, a "response" or "positive response" to the anti-cancer chemotherapy as described herein shall relate to a cancer in a patient that can be treated by providing the anti-cancer chemotherapy as described herein. Consequently, a lack of the expression of P2X4 receptor protein allows to predict no or an adverse response of said patient to the present combination chemotherapy. A positive response further may refer to a desired outcome of the therapy when applied to a patient, ideally a complete recovery from the disease, relief of symptoms or slowing down of disease progression. In the specific case of a solid tumor, a positive response preferably refers to shrinkage in tumor size or slowing down of tumor growth.

The information regarding the prediction of the response of a patient to an anti-cancer chemotherapy as described herein then allows the attending physician to adjust the treatment strategy for the patient or a group of patients accordingly (see also below).

As mentioned above, it was surprisingly found in the context of the present invention, that the expression of the P2X4 receptor protein differs significantly between cancer subtypes in patients that are responders or non-responders to the anti-cancer chemotherapy as described herein. In line with this finding, the present invention provides a method for identifying a responder to an anti-cancer chemotherapy, comprising the steps of detecting the expression of P2X4 receptor protein in cancer cells in a cancer sample obtained from a patient treated with an anti-cancer chemotherapy according to the present invention as described herein, i.e., comprising the administration of a combination of i) an effective amount of at least one inhibitor of the P2X4 receptor and/or inhibitor of the P2X4 receptor signaling pathway and ii) an effective amount of at least one cell death inducing chemotherapeutic drug and/or cell death inducing therapy.

The detection of the P2X4 receptor protein expression in the sample when compared to a control cancer sample identifies the patient as a responder to said combination chemotherapy. In the context of the present invention, a "responder" to the anti-cancer chemotherapy as described herein shall relate to a patient that can be treated by providing the anti-cancer chemotherapy as described herein. Consequently, a "non-responder" shall relate to a patient that does not react to the treatment using the anti-cancer chemotherapy as described herein.

In the context of the above aspects, the present invention also allows to monitor the treatment and/or response of the patient treated by providing the anti-cancer chemotherapy as described herein. For this, the methods of the present invention are performed with samples that have been obtained from the patient or a group of patients before and/or during the treatment with the anti-cancer chemotherapy as described herein. The information regarding the response of a cancer cell or tissue and thus the patient to the anti-cancer chemotherapy as described herein then allows the attending physician to adjust the treatment strategy for the patient or a group of patients accordingly (see also below).

Both the method for predicting the response of a patient to an anti-cancer chemotherapy as described herein and the method for identifying a responder to an anti-cancer chemotherapy as described herein allow the attending physician to adjust the treatment strategy for the patient or a group of patients. Therefore, in a preferred aspect thereof, the methods according to the present invention further comprise the step of stratifying the patient into a treatment group for applying a suitable anti-cancer therapy, in particular a combination chemotherapy comprising the administration of a combination of i) an effective amount of at least one inhibitor of the P2X4 receptor and/or inhibitor of the P2X4 receptor signaling pathway and ii) an effective amount of at least one cell death inducing chemotherapeutic drug and/or cell death inducing therapy according to the present invention.

In the context of the present invention, patients are "stratified" by distributing the patients into subgroups for treatment by the anti-cancer chemotherapy as described herein, if the treatment was identified as positive for the patient. In the context of the present invention, stratification may be further based on other clinical parameters, like gender or age or stage of the cancer patient. Furthermore, the stratification may be additionally performed based on the combination as provided.

Nevertheless, in another a preferred aspect of the method according to the present invention, the anti-cancer combination chemotherapy is a personalized combination chemotherapy for the individual patient.

As mentioned above, the method according to the present invention comprises the detection of the P2X4 receptor protein expression in a cancer cell, a cancer tissue sample comprising the cell, an organoid comprising the cancer cell or other suitable cancer sample. In general, any suitable method for detection of the P2X4 receptor protein expression may be used, nevertheless, the present data indicate that transcriptomic analyses seem not suitable to determine changes in P2X4 expression. So far, only an immunohistochemical analysis allowed for proper evaluation of the P2X4 receptor protein expression as a basis for potential cancers, responders, and a patient stratification amenable to a combination therapy.

Therefore, preferred is the method according to the present invention, wherein said determining comprises performing a protein detection method selected from the group consisting of optical methods, such as UV absorption, **bicinchoninic acid (BCA)**, Bradford assay, fluorescence-based methods, spectroscopic methods, mass spectrometry methods, and antibody-binding based methods, such as Western blotting. Further preferred is the method according to the present invention, wherein the detection comprises immunohistochemical analysis of P2X4 expression in a cancer cell, tissue or organoid.

Particularly preferred are methods based on P2X4 receptor protein antibody-antigen binding. These methods involve whole antibodies or antigen-binding fragments thereof, such as scFv fragments, or the like. More preferably, the binding moieties are suitably labelled, such as with fluorescent groups, isotopes, dyes, or enzymes.

In the context of the present invention, the term "P2X4 receptor protein" shall refers to the human amino acid sequence or a functional fragment thereof having the amino acid sequence according to UniProt accession no. Q99571, with the amino acid sequence:

MAGCCAALAAFLFEYDTPRIVLIRSRKVGLMNRAVQLLILAYVIGWVFVWEKGYQE
TDSVVSSVTTKVKGVAVTNTSKLGFRIWDVADYVIPAQEENSLFVMTNVILTMNQTQ

GLCPEIPDATTVCKSDASCTAGSAGTHSNGVSTGRCVAFNGSVKTCEVAAWCPVED
DTHVPQPAFLKAAENFTLLVKNNIWYPKFNFSKRNILPNITTTYLKSCIYDAKTDPFCP
IFRLGKIVENAGHSFQDMAVEGGIMGIQVNWDCNLDRAASLCLPRYSFRRLDTRDVE
HNVSPGYNFRFAKYYRDLAGNEQRTLIKAYGIRFDIIVFGKAGKFDIIPTMINIGSGLA
LLGMATVLCDIIVLYCMKKRLYYREKKYKYVEDYEQGLASELDQ (SEQ ID NO: 1).

The term shall also include mammalian homologues of the human protein, splice variants thereof and proteins with modifications, for example post-translational modifications such as phosphorylation, ubiquitination and/or acetalization.

In a more preferred embodiment of the method according to the present invention, the method further comprises the step of quantifying the P2X4 receptor protein. Such protein quantification techniques may be selected from, but are not limited to, optical detection methods such as UV absorption BCA or Bradford assay, fluorescence-based methods, spectroscopic methods such as mass spectrometry and preferably an immunohistochemical analysis.

In a particularly preferred embodiment of the method according to the present invention, said protein detection method comprises a high-throughput tissue protein analysis method, preferably selected from organoid and/or tissue microarray (TMA).

The methods according to the present invention relate to the treatment of cancers or neoplastic diseases in mammalian patients in general. Preferred are cancers or neoplastic diseases that are characterized by an expression of the P2X4 receptor protein as described herein. Preferred is a method according to the present invention, wherein the cancer is selected from the group consisting of a solid tumor or metastases thereof selected from Lrp5+ cancer stem cell-associated cancers, prostate cancer, pancreatic cancer, breast cancer, gastric cancer, liver cancer, brain cancer, lung cancer, kidney cancer, and metastases thereof, and preferably cancer with epithelial origin, selected from colorectal cancer, pancreatic ductal cancer, gastric cancer, adenocarcinoma of gastric-esophageal junction, lung adenocarcinoma, lung squamous epithelial cancer, endometrial cancer, salivary gland cancer and neuroendocrine tumors of the pancreas adenocarcinoma. In certain embodiments, the cancer can be, for example, primary or secondary (i.e. metastases) esophageal cancer, gastroesophageal junction cancer, gastroesophageal adenocarcinoma, gastric cancer, chondrosarcoma, colorectal adenocarcinoma, breast cancer, ovarian cancer, head and neck cancer, melanoma, gastric adenocarcinoma, lung cancer, pancreatic cancer, renal cell carcinoma, hepatocellular carcinoma, cervical cancer, brain tumour, multiple myeloma, leukaemia, lymphoma, prostate cancer, cholangiocarcinoma, endometrial cancer, small bowel adenocarcinoma, uterine sarcoma, or adrenocorticoid carcinoma. In certain embodiments, the cancer can be, for example, esophageal cancer, gastroesophageal junction cancer, gastroesophageal adenocarcinoma, colorectal adenocarcinoma, breast cancer, ovarian cancer, head and neck cancer, melanoma, gastric adenocarcinoma, lung cancer, pancreatic cancer, renal cell carcinoma, hepatocellular carcinoma, cervical cancer, brain tumour, multiple myeloma, leukaemia, lymphoma, prostate cancer, cholangiocarcinoma, endometrial cancer, small bowel adenocarcinoma, uterine sarcoma, or adrenocorticoid carcinoma. In certain embodiments, the cancer can be, for example, colorectal cancer. In certain embodiments, the cancer can be, for example, colorectal adenocarcinoma. In certain embodiments, the cancer can be, for example, small bowel adenocarcinoma. In certain embodiments, the cancer can be, for example, hepatocellular carcinoma. In certain embodiments, the cancer can be, for example, head and neck cancer. In certain embodiments, the cancer can be, for example, renal cell carcinoma. In certain embodiments, the cancer can be, for example, ovarian cancer. In certain embodiments, the cancer can be, for example, prostate cancer. In certain embodiments, the cancer can be, for example, lung cancer. In certain embodiments, the cancer can be, for example, uterine sarcoma. In certain embodiments, the cancer can be, for example, esophageal cancer. In certain embodiments, the cancer can be, for example, endometrial cancer. In certain embodiments, the cancer can be, for example, cholangiocarcinoma. In certain embodiments, each of the cancers can be, for example, unresectable, advanced, refractory, recurrent, or metastatic. A preferred embodiment, the cancer is of epithelial origin. A preferred cancer is a solid cancer, especially a solid cancer of epithelial origin, in particular hepatocellular carcinoma or colorectal cancer.

Another aspect of the present invention provides the use of a kit comprising a P2X4 receptor protein binding antibody or P2X4 receptor protein fragment or derivative thereof for identifying a cancer cell as being sensitive to an anti-cancer chemotherapy, for predicting the response of a patient to an anti-cancer chemotherapy, for identifying a responder to an anti-cancer chemotherapy and/or for stratifying a patient into a treatment group according to a method according to the present invention, and optionally further comprising materials to perform said method, such as buffers and reagents, as well as user instructions. Particularly preferred are kits comprising materials for a use based on P2X4 receptor protein antibody-antigen binding. These uses and kits involve whole antibodies or antigen-binding fragments and moieties thereof, such as scFv fragments, or the like. More preferably, the binding moieties are suitably labelled, such as with fluorescent groups, isotopes, dyes, or enzymes.

Another important aspect of the present invention then relates to a method for treating or preventing cancer, comprising the steps of performing the method according to the present invention, and administering a combination of i) an effective amount of at least one inhibitor of the P2X4 receptor and/or inhibitor of the P2X4 receptor signaling pathway and ii) an effective amount of at least one cell death inducing chemotherapeutic drug and/or cell death inducing therapy as described herein to the patient based on the result of the method according to the present invention.

The term "treating" or "treatment" as used herein means stabilizing or reducing an adverse symptom associated with a condition; reducing the severity of a disease symptom; slowing the rate of the progression of a disease; inhibiting or stabilizing the progression of a disease condition; or changing a metric that is associated with the disease state in a desirable way. The term "preventing" or "prevention" as used herein means the avoidance of the occurrence of an adverse symptom associated with a condition or disease before they occur.

The treatment may be performed in any suitable way. Preferred is the method according to the present invention, wherein the administration of the combination is concomitantly or sequentially. Further preferred is the method according to the present invention, wherein the anti-cancer combination chemotherapy further comprises a prior and/or concomitant anti-cancer chemotherapy, comprising for example cellular toxins, Lgr5 inhibitors, Lgr5+ cancer cell ablation, mTOR inhibitors, and IMPDH inhibitors.

Regardless of the route of administration selected, the compounds/agents of the anti-cancer combination chemotherapy of the present invention, which may be used in a suitable hydrated form, and/or the pharmaceutical compositions of the present invention, are formulated into pharmaceutically acceptable dosage forms by conventional methods known to those of skill in the art. Actual dosage levels of the active ingredients in the pharmaceutical compositions as used may be varied so as to obtain an amount of the active ingredient, which is effective to achieve the desired therapeutic response for a particular patient, composition, and mode of administration, without being toxic to the patient. The selected dosage level will depend upon a variety of pharmacokinetic factors including the activity of the particular compositions of the present invention employed, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts.

The dosage regimen will be determined by the attending physician and clinical factors. As is well known in the medical arts, dosages for any one patient depend upon many factors, including the patient's size, body surface area, age, the particular compound/agent to be administered, sex, time and route of administration, general health, and other drugs being administered concurrently. A typical dose can be, for example, in the range of 0.001 to 1000 µg; however, doses below or above this exemplary range are envisioned, especially considering the aforementioned factors. Generally, the regimen as a regular administration of the pharmaceutical composition should be in the range of 1 µg to 10 mg units per day. If the regimen is a continuous infusion, it should also be in the range of 1 µg to 10 mg units per kilogram of body weight per minute, respectively. Progress can be monitored by periodic assessment. The compositions of the invention may be administered locally or systemically. Administration will generally be parenterally, e.g., intravenously; the pharmaceutical composition may also be administered directly to the target site, e.g., by biolistic delivery to an internal or external target site or by catheter to a site in an artery. Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like. Furthermore, the pharmaceutical composition as used in the present invention may comprise further agents such as interleukins or interferons depending on the intended use of the pharmaceutical composition.

In the context of the present invention, P2X4 inhibitor or P2X4 receptor inhibitor shall refer to inhibitor either of the P2X4 receptor protein itself or of the P2X4 receptor signalling pathway.

P2X4 inhibitor should be understood to refer to any compound that when brought into contact with a cell inhibit the biological function of the P2X4 receptor, in particular the purinergic signaling thereof in the context of a tumor or tumor environment. Quite a few compounds have been described to have an activity as inhibitor of the P2X4 receptor and/or inhibitor of the P2X4 receptor signaling pathway, and such compounds, known to the skilled person, shall be encompassed by the present invention.

In general, any suitable at least one inhibitor of the P2X4 receptor and/or inhibitor of the P2X4 receptor signaling pathway may be used. Preferred is a method according to the present invention, wherein the inhibitor of the P2X4 receptor is a specific inhibitor of the P2X4 receptor, and is preferably selected from the group consisting of PPADS, Suramin, KN-62, TNP-ATP, Brilliant Blue G, 5-BDBD, BX-430, Carbamazepine der., PSB-12054, PSB-12062, PSB-15417, NP-1815-PX, NC-2600, UoS14919, Paroxetine, Duloxetine, BAY-1797, BAY-2328065, IgG#151-LO, an anti-P2X4 receptor protein antibody or an inhibitor binding fragment or derivative thereof, an siRNA, an shRNA, an miRNA, a ribozyme, an aptamer, an antisense nucleic acid molecule, and a small molecule of less that about 500 Da, preferably 5DBDB, and further preferred BAY-2328065.

In general, the method according to the present invention may involve any suitable cell death inducing chemotherapeutic drug and/or a cell death inducing therapy, as long as it can be combined (either provided together with, jointly, or separately as long as functioning together in the subject) with the above inhibitor of the P2X4 receptor inhibitor of the components of the P2X4 receptor signaling pathway. Further preferred is a method according to the present invention, wherein the cell death inducing chemotherapeutic drug and/or cell death inducing therapy is selected from the group consisting of necrotic cell supernatants; cationic amphiphilic drugs (CAD); classical anticancer agents, including anthracyclines, antimetabolites, and platinum drugs; topoisomerase inhibitors, e.g. camptothecin; PDE3A inhibitors, such as anagrelide, either alone or with cell death-inducing cytokines; death-inducing cytokines, such as IFN-α, IFN-γ, TNF-α, or TRAIL; taxanes, paclitaxel, and fluorinated taxanes, such as SB-T-12851, SB-T-12852, SB-T-12853, SB-T-12854, 5-fluorouracil, tegafur, capecitabine, and doxifluridine; and preferably 5-fluorouracil (5-FU).

Preferred is the method according to the present invention, wherein said combination is administered to the patient simultaneously or separately, as combined and/or separate dosage forms.

Preferred is the method according to the present invention, wherein said combination chemotherapy comprises the inhibition of the ATP-dependent P2X4 receptor- and/or P2X4 receptor signaling pathway-mediated tumor escape mechanism.

Preferred is a method according to the present invention for using the inhibitor of the P2X4 receptor or inhibitor of the P2X4 receptor signaling pathway in combination with the cell death inducing chemotherapeutic drug and/or a cell death inducing therapy, wherein said treatment and/or prevention further comprises a prior and/or concomitant anti-cancer chemotherapy, comprising for example cellular toxins, Lgr5 inhibitors, Lgr5+ cancer cell ablation, mTOR inhibitors, and IMPDH inhibitors. In general, any suitable anti-cancer chemotherapy can be used in the context of the present invention, as long as it can be combined (either provided together with, jointly, or separately as long as functioning together in the subject) with the above combination of the present invention, which effectively blocks the cancer escape-mechanism as disclosed herein, and with it a further growth of the tumor and/or the formation of metastases or a relapse. Preferred is a method for a treatment according to the present invention, said treatment comprising an inhibitor of the P2X4 receptor or inhibitor of the P2X4 receptor signaling pathway in combination with the fluoropyrimidine drug, wherein said combination is provided simultaneously or separately, as combined and/or separate dosage forms.

The methods according to the present invention relate to the treatment of cancers or neoplastic diseases in mammalian patients in general. Preferred are cancers or neoplastic diseases that are characterized by an expression of the P2X4 receptor protein as described herein. Preferred is a method according to the present invention, wherein the cancer is selected from the group consisting of a solid tumor or metastases thereof selected from Lrp5+ cancer stem cell-associated cancers, prostate cancer, pancreatic cancer, breast cancer, gastric cancer, liver cancer, brain cancer, lung cancer, kidney cancer, and metastases thereof, and preferably cancer with epithelial origin, selected from colorectal cancer, pancreatic ductal cancer, gastric cancer, adenocarcinoma of gastric-esophageal junction, lung adenocarcinoma, lung squamous epithelial cancer, endometrial cancer, salivary gland cancer and neuroendocrine tumors of the pancreas adenocarcinoma. In certain embodiments, the cancer can be, for example, primary or secondary (i.e. metastases) esophageal cancer, gastroesophageal junction cancer, gastroesophageal adenocarcinoma, gastric cancer, chondrosarcoma, colorectal adenocarcinoma, breast cancer, ovarian cancer, head and neck cancer, melanoma, gastric adenocarcinoma, lung cancer, pancreatic cancer, renal cell carcinoma, hepatocellular carcinoma, cervical cancer, brain tumour, multiple myeloma, leukaemia, lymphoma, prostate cancer, cholangiocarcinoma, endometrial cancer, small bowel adenocarcinoma, uterine sarcoma, or adrenocorticoid carcinoma. In certain embodiments, the cancer can be, for example, esophageal cancer, gastroesophageal junction cancer, gastroesophageal adenocarcinoma, colorectal adenocarcinoma, breast cancer, ovarian cancer, head and neck cancer, melanoma, gastric adenocarcinoma, lung cancer, pancreatic cancer, renal cell carcinoma, hepatocellular carcinoma, cervical cancer, brain tumour, multiple myeloma, leukaemia, lymphoma, prostate cancer, cholangiocarcinoma, endometrial cancer, small bowel adenocarcinoma, uterine sarcoma, or adrenocorticoid carcinoma. In certain embodiments, the cancer can be, for example, colorectal cancer. In certain embodiments, the cancer can be, for example, colorectal adenocarcinoma. In certain embodiments, the cancer can be, for example, small bowel adenocarcinoma. In certain embodiments, the cancer can be, for example, hepatocellular carcinoma. In certain embodiments, the cancer can be, for example, head and neck cancer. In certain embodiments, the cancer can be, for example, renal cell carcinoma. In certain embodiments, the cancer can be, for example, ovarian cancer. In certain embodiments, the cancer can be, for example, prostate cancer. In certain embodiments, the cancer can be, for example, lung cancer. In certain embodiments, the cancer can be, for example, uterine sarcoma. In certain embodiments, the cancer can be, for example, esophageal cancer. In certain embodiments, the cancer can be, for example, endometrial cancer. In certain embodiments, the cancer can be, for example, cholangiocarcinoma. In certain embodiments, each of the cancers can be, for example, unresectable, advanced, refractory, recurrent, or metastatic. A preferred embodiment, the cancer is of epithelial origin. A preferred cancer is a solid cancer, especially a solid cancer of epithelial origin, in particular hepatocellular carcinoma or colorectal cancer.

In one embodiment, the solid tumor or metastases thereof is selected from Lrp5+ cancer stem cell-associated cancers, prostate cancer, pancreatic cancer, breast cancer, gastric cancer, liver cancer, brain cancer, lung cancer, kidney cancer, and metastases thereof. Even more preferably, the solid tumor or metastases thereof is a cancer with epithelial origin, such as colorectal cancer, pancreatic ductal cancer, gastric cancer, adenocarcinoma of gastric-esophageal junction, lung adenocarcinoma, lung squamous epithelial cancer, endometrial cancer, salivary gland cancer and neuroendocrine tumors.

The present invention relates to the following items.

Item 1. A method for identifying a cancer cell as being sensitive to an anti-cancer chemotherapy comprising the administration of a combination of i) an effective amount of at least one inhibitor of the P2X4 receptor and/or inhibitor of the P2X4 receptor signaling pathway and ii) an effective amount of at least one cell death inducing chemotherapeutic drug and/or cell death inducing therapy, comprising the step of detecting P2X4 receptor protein expression in a cancer cell, wherein the detection of P2X4 receptor protein expression in the cancer cell when compared to a non-P2X4 receptor protein expressing cancer control cell identifies the cancer cell as being sensitive to said combination chemotherapy.

Item 2. A method for predicting the response of a patient to an anti-cancer chemotherapy comprising the administration of a combination of i) an effective amount of at least one inhibitor of the P2X4 receptor and/or inhibitor of the P2X4 receptor signaling pathway and ii) an effective amount of at least one cell death inducing chemotherapeutic drug and/or cell death inducing therapy, said method comprising the step of detecting the expression of P2X4 receptor protein in cancer cells in a cancer sample from said patient, wherein the detection of P2X4 receptor protein expression in the sample when compared to a non-P2X4 receptor protein expressing cancer control cell predicts a positive response of said patient to said combination chemotherapy.

Item 3. A method for identifying a responder to an anti-cancer chemotherapy, comprising the steps of detecting the expression of P2X4 receptor protein in cancer cells in a cancer sample obtained from a patient treated with an anti-cancer chemotherapy comprising the administration of a combination of i) an effective amount of at least one inhibitor of the P2X4 receptor and/or inhibitor of the P2X4 receptor signaling pathway and ii) an effective amount of at least one cell death inducing chemotherapeutic drug and/or cell death inducing therapy, wherein the detection of the P2X4 receptor protein expression in the sample when compared to a control cancer sample identifies the patient as a responder to said combination chemotherapy.

Item 4. The method according to Item 2 or 3, further comprising the step of stratifying the patient into a treatment group for applying a suitable anti-cancer therapy, in particular a combination chemotherapy comprising the administration of a combination of i) an effective amount of at least one inhibitor of the P2X4 receptor and/or inhibitor of the P2X4 receptor signaling pathway and ii) an effective amount of at least one cell death inducing chemotherapeutic drug and/or cell death inducing therapy.

Item 5. The method according to any one of Items 1 to 4, wherein the administration of the combination is concomitantly or sequentially.

Item 6. The method according to any one of Items 1 to 5, wherein the detection of the P2X4 receptor protein expression comprises determining the protein expression of the P2X4 receptor protein in the cancer cell, a cancer tissue sample comprising the cell, an organoid comprising the cancer cell or other suitable cancer sample.

Item 7. The method according to Item 6, wherein said determining comprises performing a protein detection method selected from the group consisting of optical methods, such as UV absorption, **bicinchoninic acid (BCA)**, Bradford assay, fluorescence-based methods, spectroscopic methods, mass spectrometry methods, and antibody-binding based methods, such as Western blotting.

Item 8. The method according to Item 6 or 7, wherein the detection comprises immunohistochemical analysis of P2X4 expression in a tissue or organoid.

Item 9. The method according to any one of Items 6 to 8, further comprising the step of quantifying the P2X4 receptor protein.

Item 10. The method according to any one of Items 6 to 9, wherein the method is a high-throughput method, preferably comprising a tissue microarray (TMA).

Item 11. The method according to any one of Items 1 to 10, wherein the cancer is selected from the group consisting of a solid tumor or metastases thereof selected from Lrp5+ cancer stem cell-associated cancers, prostate cancer, pancreatic cancer, breast cancer, gastric cancer, liver cancer, brain cancer, lung cancer, kidney cancer, and metastases thereof, and preferably cancer with epithelial origin, selected from colorectal cancer, pancreatic ductal cancer, gastric cancer, adenocarcinoma of gastric-esophageal junction, lung adenocarcinoma, lung squamous epithelial cancer, endometrial cancer, salivary gland cancer and neuroendocrine tumors of the pancreas adenocarcinoma.

Item 12. The method according to any one of Items 1 to 11, wherein the inhibitor of the P2X4 receptor is a specific inhibitor of the P2X4 receptor, and is preferably selected from the group consisting of PPADS, Suramin, KN-62, TNP-ATP, Brilliant Blue G, 5-BDBD, BX-430, Carbamazepine der., PSB-12054, PSB-12062, PSB-15417, NP-1815-PX, NC-2600, UoS14919, Paroxetine, Duloxetine, BAY-1797, BAY-2328065, IgG#151-LO, an antibody or an inhibitor binding fragment or derivative thereof, an siRNA, an shRNA, an miRNA, a ribozyme, an aptamer, an antisense nucleic acid molecule, and a small molecule of less that about 500 Da, preferably 5DBDB, and further preferred BAY-2328065.

Item 13. The method according to any one of Items 1 to 12, wherein the cell death inducing chemotherapeutic drug is selected from the group consisting of necrotic cell supernatants; cationic amphiphilic drugs (CAD); classical anticancer agents, including anthracyclines, antimetabolites, and platinum drugs; topoisomerase inhibitors, e.g. camptothecin; PDE3A inhibitors, such as anagrelide, either alone or with cell death-inducing cytokines; death-inducing cytokines, such as IFN-α, IFN-γ, TNF-α, or TRAIL; taxanes, paclitaxel, and fluorinated taxanes, such as SB-T-12851, SB-T-12852, SB-T-12853, SB-T-12854, 5-fluorouracil, tegafur, capecitabine, and doxifluridine; and preferably 5-fluorouracil (5-FU).

Item 14. The method according to any one of Items 1 to 13, wherein the anti-cancer combination chemotherapy further comprises a prior and/or concomitant anti-cancer chemotherapy, comprising for example cellular toxins, Lgr5 inhibitors, Lgr5+ cancer cell ablation, mTOR inhibitors, and IMPDH inhibitors.

Item 15. The method according to any one of Items 1 to 14, wherein the anti-cancer combination chemotherapy is a personalized combination chemotherapy for the patient.

Item 16. Use of a kit comprising a P2X4 receptor protein binding antibody or P2X4 receptor protein fragment or derivative thereof for identifying a cancer cell as being sensitive to an anti-cancer chemotherapy, for predicting the response of a patient to an anti-cancer chemotherapy, for identifying a responder to an anti-cancer chemotherapy and/or for stratifying a patient into a treatment group according to a method according to any one of Items 1 to 15, and optionally further comprising materials to perform said method, such as buffers and reagents, as well as user instructions.

Item 17. A method for treating cancer, comprising the steps of performing the method according to any one of Items 1 to 15, and administering a combination of i) an effective amount of at least one inhibitor of the P2X4 receptor and/or inhibitor of the P2X4 receptor signaling pathway and ii) an effective amount of at least one cell death inducing chemotherapeutic drug and/or cell death inducing therapy to the patient based on the result of the method according to any one of Items 1 to 15.

Item 18. The method according to Item 17, wherein said combination is administered to the patient simultaneously or separately, as combined and/or separate dosage forms.

Item 19. The method according to Item 17 or 18, wherein said combination chemotherapy comprises the inhibition of the ATP-dependent P2X4 receptor- and/or P2X4 receptor signaling pathway-mediated tumor escape mechanism.

Item 20. The method according to any one of Items 17 to 19, wherein the cancer is selected from the group consisting of a solid tumor or metastases thereof selected from Lrp5+ cancer stem cell-associated cancers, prostate cancer, pancreatic cancer, breast cancer, gastric cancer, liver cancer, brain cancer, lung cancer, kidney cancer, and metastases thereof, and preferably cancer with epithelial origin, selected from colorectal cancer, pancreatic ductal cancer, gastric cancer, adenocarcinoma of gastric-esophageal junction, lung adenocarcinoma, lung squamous epithelial cancer, endometrial cancer, salivary gland cancer and neuroendocrine tumors of the pancreas adenocarcinoma.

Item 21. The method according to any one of Items 17 to 20, wherein the anti-cancer combination chemotherapy further comprises a prior and/or concomitant anti-cancer chemotherapy, comprising for example cellular toxins, Lgr5 inhibitors, Lgr5+ cancer cell ablation, mTOR inhibitors, and IMPDH inhibitors.

The present invention will now be described further in the following examples with reference to the accompanying Figures, nevertheless, without being limited thereto. For the purposes of the present invention, all references as cited herein are incorporated by reference in their entireties.

**Figure 1** shows an analysis of P2RX4 in the four consensus molecular subtypes of CRC according to GSE39582 (TCGA): CMS 1 (n=72), CMS2 (n=162), CMS3 (n=73), CMS4 (n=127).

**Figure 2** shows an overview of immunohistochemical analysis of P2X4 expression in the respective tumor entities. Expression was scored as 0, +, ++ or +++.

**Figure 3** shows the immunoblot analysis of P2X4 in SU-DHL5 lymphoma cells and three human colorectal cancer organoids (A). Survival of SU-DHL5 cells in response to doxorubicin in the absence or presence of 5-BDBD (B). Survival of human CRC tumor organoids in response to 5-FU in the absence or presence of 5-BDBD (C).

### Examples

### Transcriptomic analysis of the P2RX4 gene

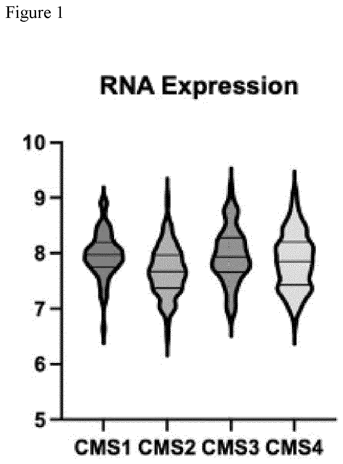

Based on previous data of the inventors indicating that chemotherapy-induced cell death in colorectal and pancreatic cancer induces a P2X4/mTOR dependent vulnerability in neighboring epithelial cells that renders these tumor sensitive to a combination therapy (chemotherapy plus targeting of P2X4; Schmitt et al., Nature, 2022), the inventors now evaluated whether changes in the P2RX4 gene could be associated with a particular molecular subtype of colorectal cancer. However, differences in P2RX4 gene expression between the different colorectal cancer subtypes were not significant (Fig. 1).

### Immunohistochemical analysis of P2X4 protein level

The inventors then examined whether P2X4 expression may differ on the protein level in colorectal cancer and further extended the analysis to other tumor entities originating in epithelia that are known to express P2X4 under homeostatic conditions.

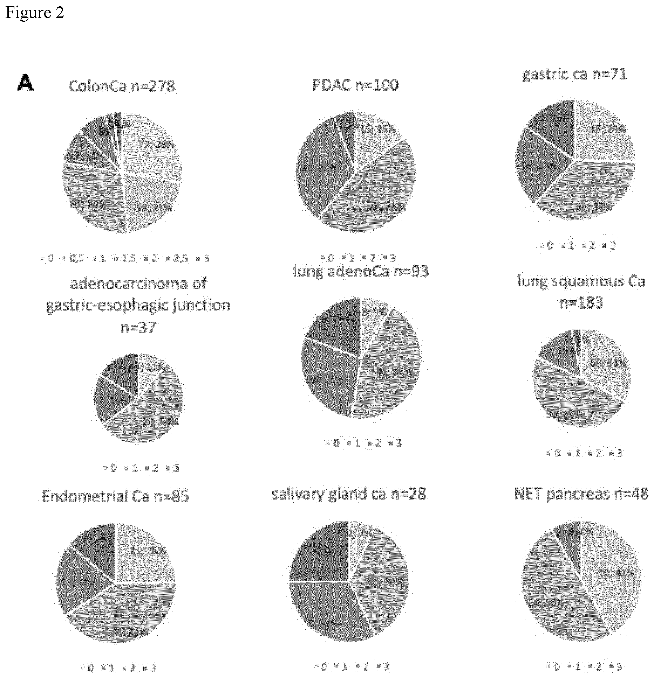

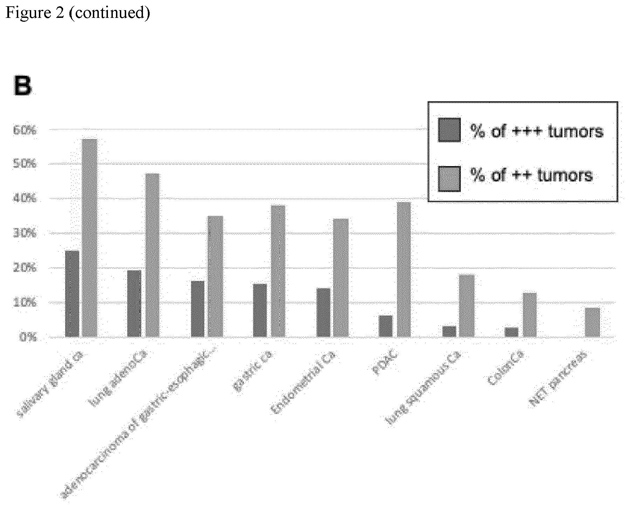

To this end the inventors performed an immunohistochemical analysis of P2X4 employing tissue microarrays (TMA) of colon cancer (n=278), pancreatic ductal cancer (n=100), gastric cancer (n=71 tumors), adenocarcinoma of gastric-esophagic junction (n=37), lung adenocarcinoma (n=93), lung squamous epithelial cancer (n=183), endometrial cancer (n=85), salivary gland cancer (n=28), and neuroendocrine tumors of the pancreas (n=48). In all tumor entities analyzed, the inventors found a very heterogenous pattern of P2X4 expression ranging from complete lack of expression to strong expression (Fig. 2).

Differences in P2X4 expression are very likely associated with a better or worse prognosis. In summary, the present data indicate that transcriptomic analyses seem not suitable to determine changes in P2X4 expression. So far, only an immunohistochemical analysis allowed for proper evaluation of potential cancers, responders, and a patient stratification to a combination therapy.

### Immunoblot analysis revealed that only P2X4 expressing tumor cells are sensitive to combination therapy

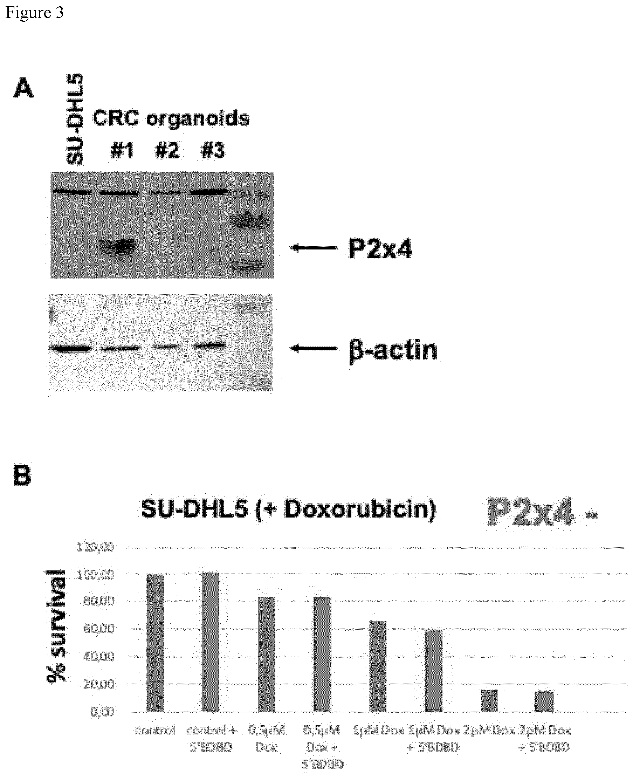

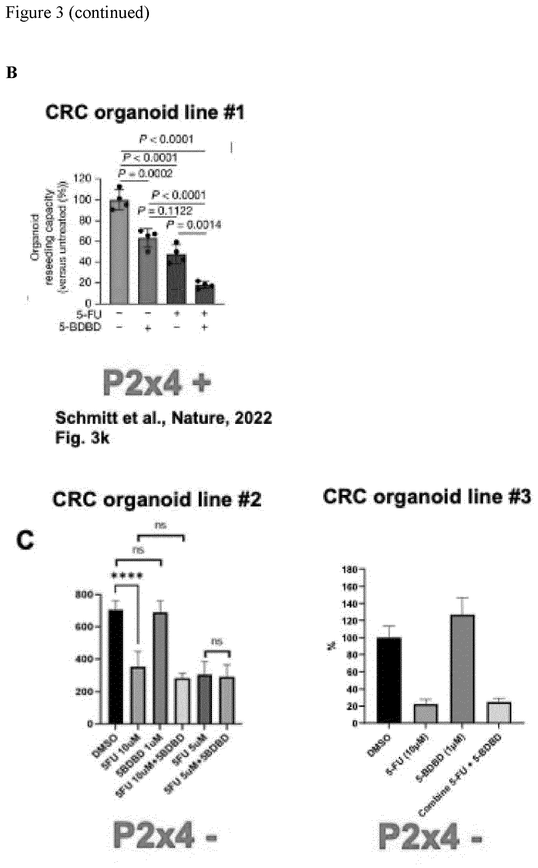

To ultimately show that indeed only P2X4 expressing tumor cells are sensitive to combination therapy, the inventors compared the response to 5-FU +/- P2X4 inhibitor in colorectal cancer organoids that either expressed P2X4 or not. The inventors had previously shown that blocking P2X4 would sensitize organoids to 5-FU treatment (Schmitt et al, Nature, 2022). The inventors now confirmed that these organoids indeed express P2X4 (Fig. 3A, CRC-organoid #1). In stark contrast, in colorectal cancer organoids as well as lymphoma cells that do not express P2X4 protein, no sensitization towards P2X4 inhibition could be observed (Fig. 3B, C). This occurred despite significant cell death induction upon chemotherapy, while additional P2X4 inhibition did not have any impact. Collectively, the data clearly prove that a combination therapy (chemotherapy plus P2X4 inhibitor) will only be beneficial for the treatment of patients with tumors that display tumor cell specific P2X4 expression, and that such patients can be identified by immunohistochemical analysis of P2X4 in primary tumor tissue and/or metastases.

## Claims

1. A method for identifying a cancer cell as being sensitive to an anti-cancer chemotherapy comprising the administration of a combination of i) an effective amount of at least one inhibitor of the P2X4 receptor and/or inhibitor of the P2X4 receptor signaling pathway and ii) an effective amount of at least one cell death inducing chemotherapeutic drug and/or cell death inducing therapy, comprising the step of detecting P2X4 receptor protein expression in a cancer cell, wherein the detection of P2X4 receptor protein expression in the cancer cell when compared to a non-P2X4 receptor protein expressing cancer control cell identifies the cancer cell as being sensitive to said combination chemotherapy.

2. A method for predicting the response of a patient to an anti-cancer chemotherapy comprising the administration of a combination of i) an effective amount of at least one inhibitor of the P2X4 receptor and/or inhibitor of the P2X4 receptor signaling pathway and ii) an effective amount of at least one cell death inducing chemotherapeutic drug and/or cell death inducing therapy, said method comprising the step of detecting the expression of P2X4 receptor protein in cancer cells in a cancer sample from said patient, wherein the detection of P2X4 receptor protein expression in the sample when compared to a non-P2X4 receptor protein expressing cancer control cell predicts a positive response of said patient to said combination chemotherapy, preferably further comprising the step of stratifying the patient into a treatment group for applying a suitable anti-cancer therapy, in particular a combination chemotherapy comprising the administration of a combination of i) an effective amount of at least one inhibitor of the P2X4 receptor and/or inhibitor of the P2X4 receptor signaling pathway and ii) an effective amount of at least one cell death inducing chemotherapeutic drug and/or cell death inducing therapy.

3. A method for identifying a responder to an anti-cancer chemotherapy, comprising the steps of detecting the expression of P2X4 receptor protein in cancer cells in a cancer sample obtained from a patient treated with an anti-cancer chemotherapy comprising the administration of a combination of i) an effective amount of at least one inhibitor of the P2X4 receptor and/or inhibitor of the P2X4 receptor signaling pathway and ii) an effective amount of at least one cell death inducing chemotherapeutic drug and/or cell death inducing therapy, wherein the detection of the P2X4 receptor protein expression in the sample when compared to a control cancer sample identifies the patient as a responder to said combination chemotherapy, preferably further comprising the step of stratifying the patient into a treatment group for applying a suitable anti-cancer therapy, in particular a combination chemotherapy comprising the administration of a combination of i) an effective amount of at least one inhibitor of the P2X4 receptor and/or inhibitor of the P2X4 receptor signaling pathway and ii) an effective amount of at least one cell death inducing chemotherapeutic drug and/or cell death inducing therapy.

4. The method according to any one of claims 1 to 3, wherein the administration of the combination is concomitantly or sequentially.

5. The method according to any one of claims 1 to 4, wherein the detection of the P2X4 receptor protein expression comprises determining the protein expression of the P2X4 receptor protein in the cancer cell, a cancer tissue sample comprising the cell, an organoid comprising the cancer cell or other suitable cancer sample, wherein preferably said determining comprises performing a protein detection method selected from the group consisting of optical methods, such as UV absorption, bicinchoninic acid (BCA), Bradford assay, fluorescence-based methods, spectroscopic methods, mass spectrometry methods, and antibody-binding based methods, such as Western blotting.

6. The method according to claim 4 or 5, wherein the detection comprises immunohistochemical analysis of P2X4 expression in a tissue or organoid.

7. The method according to any one of claims 4 to 6, further comprising the step of quantifying the P2X4 receptor protein.

8. The method according to any one of claims 4 to 7, wherein the method is a high-throughput method, preferably comprising a tissue microarray (TMA).

9. The method according to any one of claims 1 to 8, wherein the cancer is selected from the group consisting of a solid tumor or metastases thereof selected from Lrp5+ cancer stem cell-associated cancers, prostate cancer, pancreatic cancer, breast cancer, gastric cancer, liver cancer, brain cancer, lung cancer, kidney cancer, and metastases thereof, and preferably cancer with epithelial origin, selected from colorectal cancer, pancreatic ductal cancer, gastric cancer, adenocarcinoma of gastric-esophageal junction, lung adenocarcinoma, lung squamous epithelial cancer, endometrial cancer, salivary gland cancer and neuroendocrine tumors of the pancreas adenocarcinoma.

10. The method according to any one of claims 1 to 9, wherein the inhibitor of the P2X4 receptor is a specific inhibitor of the P2X4 receptor, and is preferably selected from the group consisting of PPADS, Suramin, KN-62, TNP-ATP, Brilliant Blue G, 5-BDBD, BX-430, Carbamazepine der., PSB-12054, PSB-12062, PSB-15417, NP-1815-PX, NC-2600, UoS14919, Paroxetine, Duloxetine, BAY-1797, BAY-2328065, IgG#151-LO, an antibody or an inhibitor binding fragment or derivative thereof, an siRNA, an shRNA, an miRNA, a ribozyme, an aptamer, an antisense nucleic acid molecule, and a small molecule of less that about 500 Da, preferably 5-DBDB, further preferred BAY-2328065, and/or wherein the cell death inducing chemotherapeutic drug is selected from the group consisting of necrotic cell supernatants; cationic amphiphilic drugs (CAD); classical anticancer agents, including anthracyclines, antimetabolites, and platinum drugs; topoisomerase inhibitors, e.g. camptothecin; PDE3A inhibitors, such as anagrelide, either alone or with cell death-inducing cytokines; death-inducing cytokines, such as IFN-α, IFN-γ, TNF-α, or TRAIL; taxanes, paclitaxel, and fluorinated taxanes, such as SB-T-12851, SB-T-12852, SB-T-12853, SB-T-12854, 5-fluorouracil, tegafur, capecitabine, and doxifluridine; and preferably 5-fluorouracil (5-FU).

11. The method according to any one of claims 1 to 10, wherein the anti-cancer combination chemotherapy further comprises a prior and/or concomitant anti-cancer chemotherapy, comprising for example cellular toxins, Lgr5 inhibitors, Lgr5+ cancer cell ablation, mTOR inhibitors, and IMPDH inhibitors.

12. The method according to any one of claims 1 to 11, wherein the anti-cancer combination chemotherapy is a personalized combination chemotherapy for the patient.

13. Use of a kit comprising a P2X4 receptor protein binding antibody or P2X4 receptor protein fragment or derivative thereof for identifying a cancer cell as being sensitive to an anti-cancer chemotherapy, for predicting the response of a patient to an anti-cancer chemotherapy, for identifying a responder to an anti-cancer chemotherapy and/or for stratifying a patient into a treatment group according to a method according to any one of claims 1 to 12, and optionally further comprising materials to perform said method, such as buffers and reagents, as well as user instructions.

14. A method for treating cancer, comprising the steps of performing the method according to any one of claims 1 to 12, and administering a combination of i) an effective amount of at least one inhibitor of the P2X4 receptor and/or inhibitor of the P2X4 receptor signaling pathway and ii) an effective amount of at least one cell death inducing chemotherapeutic drug and/or cell death inducing therapy to the patient based on the result of the method according to any one of claims 1 to 12, wherein preferably the combination is administered to the patient simultaneously or separately, as combined and/or separate dosage forms, and/or wherein said combination chemotherapy comprises the inhibition of the ATP-dependent P2X4 receptor- and/or P2X4 receptor signaling pathway-mediated tumor escape mechanism.

15. The method according to claim 14, wherein the anti-cancer combination chemotherapy further comprises a prior and/or concomitant anti-cancer chemotherapy, comprising for example cellular toxins, Lgr5 inhibitors, Lgr5+ cancer cell ablation, mTOR inhibitors, and IMPDH inhibitors.
